# EUROPEAN PATENT SPECIFICATION

(11) **EP 2 997 372 B1**
(45) Date of publication and mention of the grant of the patent: **19.06.2019**
(21) Application number: 14736473.1
(22) Date of filing: 13.05.2014
(51) Int. Cl.: G01N 33/543, C07K 14/44

(54) **BIOCHIP, ANTIGEN BOUQUET, OPTICAL READER AND METHOD FOR DETECTING AND MONITORING DISEASES**
BIOCHIP, ANTIGENBÜNDEL, OPTISCHER LESER UND VERFAHREN ZUR ERKENNUNG UND ÜBERWACHUNG VON ERKRANKUNGEN
BIOPUCE, BOUQUET D'ANTIGÈNES, LECTEUR OPTIQUE ET PROCÉDÉ DE DÉTECTION ET DE SURVEILLANCE DE MALADIES

(30) Priority: 15.05.2013 PT 10694213
(43) Date of publication of application: 23.03.2016
(73) Proprietor: Stab Vida - Investigação e Serviços em Ciências Biológicas Lda, CEP : 2825-182 Caparica (PT); BCA-clinic Betriebs GmbH & Co. KG, 86199 Augsburg (DE); Microliquid SL, 20500 Arrasate Mondragon (ES); Rational Mechanics SA, 6612 Ascona (TI) (CH)
(72) Inventor: DORMALE, Guerric Meurice de, 1435 Hevillers (BE); DEMARTEAU, Jean, 5101 Erpent (BE); WHITLOW, Harry J., Lafayette, LA 70504 (US); GILBERT, Leona, 40520 Jyväskylä (FI); PUTTARAKSA, Nitipon, 40700 Jyväskylä (FI); GARCIA-NOGALES, Paula, 08029 Barcelona (ES)
(74) Representative: Pereira Garcia, João Luís
(86) International application number: PCT/PT2014/000030
(87) International publication number: WO 2014/185803

(56) References cited:
- WO-A2-2007/033385
- US-A1- 2006 194 267
- US-A1- 2006 194 267
- US-A1- 2012 142 023
- US-A1- 2012 142 023
- US-A1- 2012 177 543
- Anonymous: "Chronic Lyme disease - Wikipedia", , 19 May 2017 (2017-05-19), XP055380461, Retrieved from the Internet: URL:https://en.wikipedia.org/wiki/Chronic_ Lyme_disease [retrieved on 2017-06-12]
- DU ET AL: "Antigen biochips verify and extend the scope of antibody detection in Lyme borreliosis", DIAGNOSTIC MICROBIOLOGY AND INFECTIOUS DISEASE, ELSEVIER, AMSTERDAM, NL, vol. 59, no. 4, 20 September 2007 (2007-09-20), pages 355-363, XP022373407, ISSN: 0732-8893, DOI: 10.1016/J.DIAGMICROBIO.2007.06.006
- Hiep T Tran ET AL: "Novel Multiplex Test for Lyme Disease", , 2 August 2010 (2010-08-02), XP055104045, Retrieved from the Internet: URL:http://www.rockland-inc.com/uploadedFi les/Support/Poster on LymeDisease_PhaseI.pdf [retrieved on 2014-02-24]
- Anonymous: "Chronic Lyme disease - Wikipedia", , 19 May 2017 (2017-05-19), XP055380461, Retrieved from the Internet: URL:https://en.wikipedia.org/wiki/Chronic_ Lyme_disease [retrieved on 2017-06-12]
- DU ET AL: "Antigen biochips verify and extend the scope of antibody detection in Lyme borreliosis", DIAGNOSTIC MICROBIOLOGY AND INFECTIOUS DISEASE, ELSEVIER, AMSTERDAM, NL, vol. 59, no. 4, 20 September 2007 (2007-09-20), pages 355-363, XP022373407, ISSN: 0732-8893, DOI: 10.1016/J.DIAGMICROBIO.2007.06.006
- Hiep T Tran ET AL: "Novel Multiplex Test for Lyme Disease", , 2 August 2010 (2010-08-02), XP055104045, Retrieved from the Internet: URL:http://www.rockland-inc.com/ [retrieved on 2014-02-24]
- BIESIADA G ET AL: "Lyme disease: Review", ARCHIVES OF MEDICAL SCIENCE 2012 TERMEDIA PUBLISHING HOUSE LTD. POL, vol. 8, no. 6, December 2012 (2012-12), pages 978-982, ISSN: 1734-1922
- "Stages of Lyme Disease - Topic Overview", , Retrieved from the Internet: URL:www.webmd.com [retrieved on 2017-10-27]
- SINGH S K ET AL: "Lyme borreliosis: from infection to autoimmunity.", CLINICAL MICROBIOLOGY AND INFECTION : THE OFFICIAL PUBLICATION OF THE EUROPEAN SOCIETY OF CLINICAL MICROBIOLOGY AND INFECTIOUS DISEASES JUL 2004, vol. 10, no. 7, July 2004 (2004-07), pages 598-614, ISSN: 1198-743X

## Description

### BACKGROUND OF THE INVENTION

### Field of the Invention

The present invention relates to a biochip comprising an antigen bouquet, to be inserted into an optical reader and relates to a method for detecting and monitoring antibodies associated with the presence of diseases in biological samples, using such biochip and such optical reader.

### Description of Prior Art

### 1 - Biochip and Antigen Bouquet

There are some documents disclosing the use of antigens and also biochips to perform a more sensitive diagnosis of some diseases, comparatively to ELISA and WB. The present invention was first directed to solve the technical problem of how to use and improve the performance of such technology and simultaneously detect autoimmunity related Lyme disease and coinfection of *B. burgdorferi* with other pathogenic bacteria, such as *Babesia microti* and *Ehrlichia.*

Some tests were already performed for detecting other diseases and the results were significantly better than with those obtained with the conventional methods or even with other biochips, antigens, optical readers and methods previously disclosed.

### Lyme Disease

Lyme disease is an endemic disease, transmitted by ticks and caused by the bacterium *Borrelia burgdorferi f.l.,* affecting 63 countries around the world, including the 27 member States of the European Union and also the United States of America.

The incidence in children is higher than in adults. The Regional Office for Europe of the World Health Organization (WHO) estimates that, currently, every year 85.000 new cases of Lyme disease arise in Europe (evaluating national data), but there is a general concern about this number being much higher, because in Europe the reported cases are highly inconsistent and many patients actually afected by this disease are not diagnosed properly in due time.

The number of reported cases of Lyme disease has increased since the early 1990s, and the geographical distribution has expanded due to climate change (*Lindgren, E. and Jaenson, Thomas G. T.,* 2006). In 1993, it was estimated that this disease had a cost of EUR 660 million for the community, being the cost per patient of approximately 40,750 euros (Irwin T. Vanderhoof, PhD, CLU et *al.,* 1993). Since then, the number of cases more than doubled, resulting in a cost increase to society in more than 1300 million euros.

The first symptoms of Lyme disease include fever, headache, fatigue, depression and a characteristic skin rash called *erythema migrans.* When detected in due time, the infection and the symptoms can be eliminated with the use of antibiotics. However, if this disease is not treated, the disease progresses and symptoms may affect the central nervous system, heart and joints, the symptoms becoming much more difficult to deal with and the disease more difficult to treat.

Occasionally, the symptoms such as arthritis persist after the infection has been eliminated, which suggests that the *Borrelia burgdorferi f.l.* may promote chronical and autoimmune induced infections (*Steer et al.* ,2004).

### Borrelia burgdorferi f.l., the bacterium causing Lyme disease

*Borrelia burgdorferi f.l.* is a pleomorphic bacterium with a complex life cycle that encompasses a multitude of forms, including the form of corkscrew (called parental, because it is the form that this bacterium has when infects the host), the form of cysts (without cell wall), among other forms. Although known for more than a century, all the bacteria with deficient cell wall (CWDB), including *Borrelia,* were only linked to clinically relevant diseases during the last few decades.
The lack of wall in this type of bacteria makes them very difficult to detect by the usual screening methods, such as the light microscope and serological pattern test. This is because this type of bacteria triggers an immune response different from the normal pattern. As a result, the CWDB creates severe difficulties in diagnosing and treating certain infections, remaining in the body for a long period of time, frequently causing chronic diseases.

The existence of pleomorphic variants of the spirochete *Borrelia* explains the ability that this bacterium has to remain in the host as dormant, during prolonged periods of latency, clinically asymptomatic. According to the symptoms and duration of the disease, there are 3 stages for Lyme disease:
1) Early stage with localized infection of the skin (*"erythema migrans"*);
2) Acute or intermediate stage wherein the infection is characterized by inflammation of various organs, such as skin, joints, heart and nervous system, weeks or even months after the transmission of *Borrelia;*
3) Chronic stage with persistent infection with chronic inflammation and decreased function of different organs and systems for more than 1 year.

The bacterium *Borrelia burgdorferi f.l.* was implicated in more severe clinical manifestations, such as borreliosis neocortical (neuroborreliosis), which has been associated with Alzheimer's disease.

### Available diagnostic methods for detecting the presence of Lyme disease

Common symptoms caused by Lyme disease symptoms are similar to a flu, headaches, pains in the joints, gastrointestinal problems, sensitivity to light/sound and a general malaise, but the variety of symptoms differs from patient to patient: some patients present only infections on the skin while others only develop late symptoms, as arthritis.
As only 60% of patients develop characteristics of *"erythema migrans"* and approximately 25-30% of patients remember to have been bitten by ticks, the remaining 10-15% of the patients are only confirmed by laboratory tests. The late stage symptoms of this disease are a result, in part, of the lack of sensitivity and efficiency of diagnostic tools that are able to detect the disease in its early stages, when it is easily treated.
The Center for Disease Control (CDC) of the United States of America proposes a criterion of diagnosis in two levels, for the serological diagnosis of Lyme disease:
A first level where it performs a positive immunological test (ELISA), followed by a second level where a Western Blot (WB) is performed to confirm the results of the immunological assay (ELISA). Both tests have lack of sensitivity and the interpretation of WB varies from laboratory to laboratory.
The main limitations of ELISA are the false positive results due to interference with an IgM rheumatoid factor and false negative IgM results due to competition with specific IgG (Hansen, 1994). Another problem with this method is to distinguish active from past infection. WB assays appears to be as sensitive and specific as ELISA but is in fact more laborious to perform, needs more sample volume and is more expensive (Carlsson et al., 1998; Magnarelli et al., 2004). The advantage of WB is that antibodies can be detected, which may recognize different antigens of a given infectious agent, and the disadvantage is the possible identification of nonspecific antigens or cross-reactive epitopes. In addition, conformational epitopes may be lost (Hansen, 1994) and WB results are also dependent on the *B. burgdorferi* strains. Therefore WB is often used to just corroborate or support results from other diagnostic methods.

This strategy therefore failed the diagnostics for the first phase of the disease in 80% of cases (Wilske, Bettina (2005), and does not distinguish between acute or chronic infection. The same problems of sensitivity are found in the evaluation of cerebroespinal fluid (CSF), where the serological test detects only 10-30% of patients with *neuroborreliosis.*
Up to date, there is no diagnostic tool able to effectively detect with sensitivity and specificity the immune response triggered by cell wall deficient forms of *Borrelia burgdorferi f.l.,* which are responsible for severe chronic symptoms of *borreliosis.* These forms have a pattern of protein expression changed, as well as differences in antigen compared with the form "parental". As a result, these forms are not detected with commercial methods based on antigens contained in total cell lysates of the "parental" form of *Borrelia burgdorferi f.l.*

### Methods for detecting and monitoring the stage of Lyme disease

In recent years, new peptides and antigenic proteins for detection of infections by *Borrelia burgdorferi f.l.* have been described in scientific and medical articles, both in the first stage of the disease and in more advanced stages. The latest research in this area points to a combination of antigens in a single test, as being the best way to overcome the problems occurring with available tests.

Two concerns arise from the available diagnosis tools and methods:
1) Detecting Lyme disease on an early stage, when it is still treatable; and
2) Distinguishing between chronic (late-stage) and acute (intermediate) infection.

Document US 2013/0273572 A1 discloses a method for diagnosing Lyme disease status in biological samples obtained or derived from a mammal, determining antibodies to *Borrelia burgdorferi* outer surface proteins (Osp) and also discloses means to identify mammals having early, intermediate or chronic B. *burgdorferi* infection. One embodiment discloses a lateral flow device comprising OspA, OspC and OspF antigens in physical association with a solid matrix. Another embodiment discloses a multiplex assay comprising the OspA, OspC and OspF antigens provided in association with fluorescent beads. The OspA, OspC and OspF antigens are contacted with a biological sample derived from an equine in a manner that antibodies to such antigens, if present, will bind to the antigen and will thereby be immobilized on the antigen/fluorescent bead complexes. A detectably labelled anti-equine specific antibody is added and a multiplex analyser is used to detect the fluorescent beads and therefore determining the status of Lyme disease.

Based on the state of the art and the available products and methods in the market, two main further requirements are clearly identifiable:
1- Providing a more effective and fast alternative to Western Blot (WB) and ELISA, each one of which require extensive laboratory work, is time consuming, difficult to standardize and provides a large amount of false negative results, wherein the provided alternative should be robust, easy to use and allow unambiguous interpretations of test results.
2- Avoiding the need for performing two tests, accomplishing a specific and reliable diagnosis in a single platform, by a single process, and allowing the unequivocal detection of early, acute and chronic infections, including new and more effective antigens.

Document Du W et al., Antigen biochips verify and extend the scope of antibody detection in Lyme borreliosis, Diagnostic Microbiology and Infectious Disease, 2007, discloses a streptavidin modified biochip-based immunodiagnosis and compares it to conventional methods such as enzyme-linked immunosorbent assay (ELISA) and WB. The diagnostics sensitivity of such coated biochips was demonstrated to be identical, and the results of ELISA and WB were confirmed. This document teaches that flagellar antigens lead to a better diagnosis and describes OspC antigen as being less sensitive to identify immunity to Lyme borreliosis (LB). Binding of antibodies to flagellar antigen or OspC8 peptide was detected by fluorescein isothiocyanate-conjugated rabbit antihuman IgM antibody and IgG antibody. Fluorescence values higher than two times over the mean value of healthy controls were interpreted as being positive for antigen-specific IgG or IgM antibodies. This document teaches that, taken together, a protein biochip, as a potential substitution of ELISA, and WB method offer the opportunity to study serum immunity in a multiplicity of patients simultaneously. It also mentions that the disclosed data stimulate the extended application of biochips by using more antigens and more specific and longer peptide epitopes.

US 2006/0194267 A1 and US 2012/0142023 A1 disclose methods for detection of Lyme disease. Tran, Kohl, Finn, Chimento and Ascoli disclose a multiplex test for Lyme disease (www.rockland-inc.com).

In response to a clear need of improving the existing diagnostic methods and with the above mentioned premises in mind, a consortium of SMEs B-C-BORRELIOSIS CENTRUM AUGSBURG BETRIEBS GMBH & CO KG (BCA), microLIQUID sl (MLD), STAB VIDA, RESEARCH AND SERVICES IN BIOLOGICAL LDA. (STAB VIDA), MICRO BIO DEVICES S. R. L (MICRO BIO), aimed to develop a novel integrated biochip technology system and method to improve clinical diagnostic, disease monitoring, and treatment of Lyme disease, by enabling specific and sensitive detection of the human serological response against Borrelia infections and ultimately in any given biological samples for detecting and monitoring the stage of other diseases.

### 2 - Biochip Optical Reader

To complement and optimize the results obtained with the biochip and the method according to the present invention, the following state of the art was identified:
Document EP1239279B1 discloses a reader for biochips, such as DNA chips and protein chips, comprising a laser for emitting an excitation light, a lens for collimating such excitation light, an optical detector for detecting fluorescent light produced by such excitation light at samples deposited on said biochip. This reader further comprises means adapted for condensing said excitation light; means for scanning the biochip with the condensed laser beam so as to reduce the influence of speckle noise.

Document "Microfluidic biochip based on SPR optical reading for the detection of Legionella pneumophila, E. De Lorenzis et al." discloses an immunosensor for highly sensitive detection of L. pneumophila using Surface Plasmon Resonance (SPR) as transducing technique. To minimize the impact of this microorganism on environmental water, an accurate procedure was required to determine the presence and its enumeration. Although there are many methods known for detection of L. pneumophila in water, such as culture method or PCR techniques, they are labour-intensive and time-consuming systems. To overcome these problems, label-free biosensors for this pathogen were considered highly desirable.

With the purpose of developing the biochip optical reader of the present invention, some requirements were enumerated as follows:
- reader needs to position accurately and in a repeatable way the reaction chambers of the biochip that holds fluorescence with respect to the optical detector,
- both biochip mechanical properties and fluorescence properties are not completely known during research about reader,
- distance between detector's optic and biochip's reaction chambers has to be very accurate (in the micrometer range),
- signal to noise ratio of fluorescence may be small and some strong fluorescence signals have to be considered as noise,
- reader must implement a robust algorithm to extract fluorescence signal from images taken by the optical detector,
- enough parameters have to be available to allow sufficient tuning of the reader during the preclinical tests,
- the user needs no specific knowledge to put the biochip in the reader and to retrieve the result of the diagnostic tests, results have to be exploitable both by an external automated system and a user,
- whole equipment has to be integrated in a "small volume".

And none of the above mentioned state of the art documents or optical readers available in the market fulfilled at the time all the desired requirements.

### SUMMARY OF THE INVENTION

The work leading to this invention has received funding from the European Union's Seventh Framework Programme (FP7/2007-2013) under grant agreement number 262411.

The object of the present invention regards an integrated system and method for detecting and monitoring the presence of antibodies associated to different stages of diseases in the presence of a given antigen bouquet comprised within a biochip to be read in an optical detector according to a specific revealing and detection method.

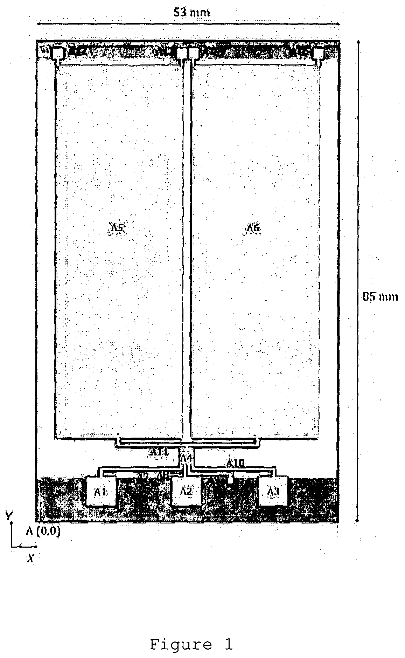

The present invention comprises a biochip with specifically designed configuration and microfluidic properties (Figure 1) that increases the speed and effectiveness of the whole process of detection and monitoring. This biochip is to be read in a specifically adapted optical reader for which an algorithm was developed and implemented to provide faster and more selective results, with a revealing method using quantum dots (qDots) which are intrinsically bright, have very narrow emission spectra, have photo-stability and are easy to use. The use of these qDots in biomedical applications is just emerging and gaining popularity. Due to their spectral characteristics the qDots have fundamental high performance in a variety of applications.

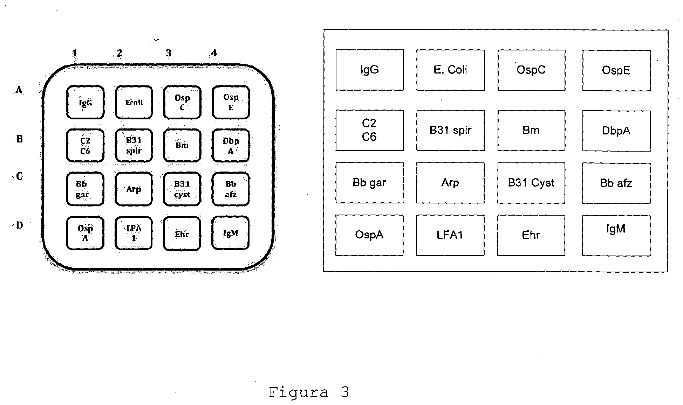

In a preferred embodiment of the present invention, it comprises an antigen bouquet with at least 16 antigen coatings (Figure 3) for primary linking to antibodies present in biological samples bearing Lyme disease and providing complexes suitable for linking specifically to secondary antibodies wherein it provides the detection of early, acute and chronic Lyme disease stages and also provides the detection of autoimmune related Lyme disease and coinfection with other antigens, other than B. burdorferi, such as *Babesia microti and Ehrlichia.*

### DESCRIPTION OF THE INVENTION

The object of the present invention was to develop a novel integrated biochip technology system and method to improve clinical diagnostic, disease monitoring, and treatment of Lyme disease, by enabling specific and sensitive detection of the human serological response against Borrelia infections and ultimately in any given biological samples for detecting and monitoring the stage of other diseases.

For that purpose, the selection of the antigenic Borrelial peptides and proteins that configure the biochip technology was a crucial step: the chronic nature of Lyme disease and the antigenic diversity of the spirochetes suggested that antigenic variation plays an important role in immune invasion.
For this reason, peptides from different components of Borrelia during different cycles were included for a best screening of Borrelia infection and other diseases.
In addition, another fundamental selection criteria was to choose peptides and proteins that have been proved to be antigens able to elicit specific serological responses in acute and chronic infections in Lyme patients. In this regard, outer surface proteins, autoimmune-related antigens, immunodominant antigens, variable major protein antigenic peptides, and proteins from decorin-binding proteins of Borrelia were tested in the present study, to evaluate their potential immunogenicity and their incorporation into the chip device. The chosen antigens and its selection reason are described as follows.

### 1. Antigen Bouquet

### Early and Acute Infection Antigens

Diagnostic laboratory tests are needed to detect active Borrelial infection, particularly early, when the hallmark clinical sign of infection, Erythema Migrans, is not seen. However, existing antibody tests require a large disease phase, often of several weeks, to become positive.
Some studies have pointed that prominent early immune responses in Lyme disease could include reactivity with the 41-kDa flagellar antigen (FlaB, p41), outer surface protein C (OspC),and **variable major protein** of the spirochete. Thus, these acute-related antigens have been chosen for testing.

It has been reported, that patients with Lyme disease usually develop IgM antibodies against the 41 kDa **flagellar antigen (p41)** of the spirochete during the early infection, followed by the development of IgG antibodies against p41 and various outer surface proteins (Osp).
Regarding Osp proteins, the 23 kDa **Outer surface protein C (OspC)** is thought to be an important virulence factor involved in the transmission and establishment of early infection. It has been shown that Borrelia burgdorferi reduces the expression of OspC in response to the development of antibodies, indicating the importance of repressing OspC expression to avoid clearance and to maintain infection. Since humans produce highly specific antibodies against OspC, measurement of IgG and IgM antibodies against this highly conserved lipoprotein indicate recent infection with B. *burgdorferi.*

Other important antigens in early Borrelia infection are **C2** and **C6 peptides.** It is well-known that the Borrelia spirochete expresses a surface lipoprotein called Variable Major protein (VMP) or Variable Major Protein Like Sequence (VIsE). VlsE plays a key role in the survival strategy of Borrelia. After penetration into the host organism, Borrelia bacteria constantly change their surface-expressed VlsE and, in this way, try to escape recognition and elimination by immune system. The VlsE protein is divided in several sections: conserved regions, which serve as transmembrane domains and anchor VlsE in the bacterial membrane, as well as variable and invariable regions. The variable regions of VlsE facing outwards are constantly changed by recombination; thus, the attacking immune system consistently encounters new altered antigen epitopes. The invariable regions are masked by the variable regions and, in living Borrelia bacteria, are protected from direct attack by the immune system. When deceased Borrelia bacteria are processed by antigen-presenting cells, the complete VlsE protein is presented to the immune system and the host also forms antibodies against the invariable and conserve regions of VlsE. These antibodies are highly suitable for diagnosis of borreliosis because of the high level of conservation of their target antigens. Among the VlsE sequence, a 19-mer (C2 peptide) and 25-mer peptide (C6 peptide) are clearly conserved across genospecies and strains of *B. burgdorferi, Sensu lato: Sensu stricto and B. gariniii.* Antigenicity of this peptide was confirmed in humans, monkeys, and mice. Sera from all these hosts reacted with C2 and C6 peptide early and persistently in the course of infection, indicating that these peptides contain one or more epitopes that are broadly antigenic against Borrelia infection.

### Chronic Infection Antigens

Most of the discovered antigens to detect Borrelia infections fit into the group of the chronic related proteins and peptides. Among them, outer surface proteins E (OspE) and A (OspA), in addition with decorin binding protein A (DbpA) have to be considered.
The ability of the Lyme spirochete to maintain chronic infection indicates that the bacteria is capable of immune evasion. The immune evasion of Lyme disease spirochetes is achieved by either antigenic variation or through the binding of Complement Regulatory Protein Factor H, avoiding complement recognition. In this mechanism of immune evasion **OspE** plays a key role:
this surface-exposed 19.2 kDa lipoprotein, expressed in both ticks and mammals, can elicit a strong antibody response. Analysis of the specificity of the antibody response to different OspE variants suggests that there are hypervariable regions that are targeted by antibody response during infection. Otherwise, there are conserved regions of OspE that are involved in Complement Regulatory Protein Factor H, facilitating complement evasion. Therefore, it is important to assess the specificity of the antibody response to OspE epitopes that are exposed during infection. For that reason, two different exposed OspE peptides will be tested in the present project: **OspE1** and **OspE2.**

Another outer surface lipoprotein that could serve as a biomarker for chronic Lyme disease is the 31 kDa **Osp A.** However, its selection criteria will be explained below (see Autoimmune Antigens section), because of its close relation with autoimmune disorders.
Regarding the decorin binding protein A (DbpA), this 19 kDa membrane protein plays a key role in the mammal colonization of the bacteria27. It has been reported to be a sensitive and a specific antigen for the serodiagnosis of Lyme arthritis or neuroborreliosis28; for this reason, this protein could be an excellent biomarker for Borrelia infection. Moreover, it has been suggested as a potential vaccine protein, due to the strong antibody response elicited during experimental murine Borreliosis. Since at the time of developing the present study, DbpA was not commercially available, the protein will be cloned, expressed and purified during the present invention's development.

### Autoimmune antigens

Long-term chronic manifestations of Lyme disease are increasingly thought to be due to induced autoimmune conditions, rather than acute pro-inflammatory action by the infection.

Antigenic mimicry of infectious agents and autoantigens is a proposed pathomechanism for autoimmune diseases. In this regard, Borrelia spirochete could be another trigger of this process where amino acid sequence homology with self- antigens may trigger multi-organ system disorder in genetically predisposed individuals31. In order to assess the presence of autoimmune disorder associated with Borrelia infection the leukocyte function-associated antigen 1 (LFA 1), the human collagen type I, the myelin basic protein (MBP), and the arthritis-related proteins GST-like self-antigen (rArp) will be include in the serological test.

A prominent late manifestation of *B. burgdorferi* infection is Lyme arthritis. Development of antibody reactivity to outer surface protein A (OspA) occurs towards the beginning of prolonged arthritic episodes. The progression to an autoimmune state begins with a crossreactive response between OspA and a self-antigen, the leukocyte function- associated antigen 1 (LFA 1). The LFA-1 is found on all T-cells and also on B-cells, macrophages and neutrophils and is involved in cell recruitment to the site of infection. During *B. burgdorferi* infection, OspA stimulates a particularly strong TH1 response in genetically susceptible individuals, which may be one of the several factors that can help to set the stage for a putative autoimmune response in affected joints33. The Identification of this cross-reactivity between OspA and LFAl may provide a model for development of autoimmune disease induced by *B. Burgdorferi,* providing then, an important antigen to incorporate into diagnostic tools.

The cross-reactivity between a Borrelia antigen and a self-antigen has been also observed in other human proteins. In Lyme disease patients, immune response against human collagen type I has been reported. It has been proposed, that this autoimmune response could be caused by cross-reactivity between an unknown Borrelia antigen (probably CRASP proteins) and human collagen type I, resulting in rheumatoid arthritis. Other example of cross-reactivity is the myelin basic protein (MBP) . Borrelia encephalomyelitis, a rare manifestation of Lyme borreliosis, may present as a multiple sclerosis (MS)-like disease. It is postulated that in MS, inflammation of the white matter is caused by a T-cell response directed to myelin antigens. In patients affected with Lyme disease, this autoimmune response could be due to a cross-reactivity between the self-antigen MBP and a Borrelia antigen, however, several investigations refused this hypothesis.

Nevertheless no matter how, both human collagen type I and MBP could be potential biomarkers for autoimmune disorders elicited by Borrelia infection.

Other potential biomarker for autoimmune disorders associated with Lyme disease is the arthritis-related proteins GST-like self-antigen (rArp). This 37 kDa arthritis-related protein of *B. burgdorferi* outer-surface protein has been shown to prevent or reduce the severity of arthritis. Antibody responses to this single protein resembling glutathione-S-Transference were tested in 124 antibiotic-treated patients with early or late manifestations of Lyme disease.

Among the 124 antibiotic-treated patients, 53% with culture-proven Erythema Migrans had IgG responses to recombinant glutathione S-transferase (GST)-Arp, as did 59% of the patients with facial palsy and 68% of those with Lyme arthritis. Therefore, measurement of antibody against recombinant GST may be an additional tool for the detection of *B. burgdorferi* involvement in patients with accompanying arthritis during initial episodes of joint pain or during the maximal period of arthritis. Similar to DbpA, Arp is not commercially available.

Thus, the protein was cloned, expressed and purified during the present development.

### Coinfection antigens

There are other organisms besides *Borrelia* that live in ticks and can be transmitted to humans through bites, causing other tick-borne illnesses. The most common ones include ***Babesia*** (babesiosis) and ***Ehrlichia*** (ehrlichiosis). *Babesia* and *Ehrlichia* are also spirochetes. The symptoms of babesiosis and ehrlichiosis often overlap those of Lyme disease and, like the last one, are just as difficult to diagnose and treat. Very often, *Babesia* and *Ehrlichia* are transmitted along with *Borrelia* by the same tick. Thus, peptides from these pathogens will be included in the study to exclude cross-reactivity with Borrelial antigens and to detect antibodies against all three spirochetes simultaneously.

Babesia species represent some of the most common infectious parasites among wild and domestic animals and are gaining increasing interest as emerging causes of zoonoses in humans. They require competent non-vertebrate and vertebrate hosts to maintain transmission cycles, infecting ixodid ticks and vertebrate erythrocytes. Several species have been shown to infect humans, although ***Babesia microti*** is the specie most frequently identified. Although the Babesia was originally identified as endemic in the Northeastern United States and parts of the Midwest, the parasite has expanded the distribution to many parts of Europe and Japan. Clinical signs in animals and in humans are not specific diagnostic measures for babesiosis, especially in asymptomatic or mixed infection in areas of endemicity.

Therefore, serological distinction of these infections is very important for choice of prophylactic treatment.

Members of the order *Rickettsiales* constitute a diverse group of intracellular bacteria of eukaryotic cells, including *Rickettsia, Orientia, Anaplasma, Ehrlichia, Neorickettsia, Bartonella* and *Coxiella.* **Ehrlichia** has been shown to be agent of human monocytic ehrlichiosis (HME) and human granulocytic ehrlichiosis (HGE). Genogroup II *Ehrlichia* express a markedly immunodominant outer membrane protein designated major surface protein 2 (MSP2). MSP2 is encoded by a multigene family, resulting in the expression of variant B cell epitopes. However, MSP2 includes conserved regions that enhances the antibody response against Ehrlichia pathogens40,41. Therefore, measurements of IgG and IgM antibody against these highly conserved regions of MSP2 are the best method for assessing humoral immune response against *Ehrlichia* pathogens.

### Bacterial Lysates

When using tests which are only based on recombinant antigens, it must be accepted that 25% of control sera usually show a false positive IgM result. For this reason, several Borrelia lysates will be included in the serological test to avoid this issue. In this regard, different genospecies of *Borrelia burgdorferi* will be evaluated for a serological response, including *sensu stricto B31, sensu stricto 297, garinii Fuji P1,* and *afzelii P12* strains.

In addition to spirochete lysates, one of the novelties of the present invention is to include cyst forms of *Borrelia burgdorferi* in the biochip test. Cystic forms (also called spheroplasts or starvation forms) represent a low metabolic activity state or phase of *B. burgdorferi* bacterial cells that allows the spirochete to survive in a hostile environment. When the Borrelia spirochete finds itself in a hostile environment it appears to change into a cyst form, covering itself with a transparent mucoid capsule; one cause of a hostile environment is antibiotics in the body serum. However, when the environment becomes favorable to its growth, the cyst opens and the spirochete is released. When in the cystic form, conventional antibiotic therapy will not destroy the Lyme spirochete. It has been reported that cyst inoculation in mice induces a specific antibody response as a sign of vigorous humoral immune response, thus indicating probable active infection. Considering this, cyst lysates was included to improve Lyme disease diagnosis.

Finally, since all the recombinant proteins commercially available or produced in the present development will be generated in *Escherichia coli,* full lysates of the **E. coll DH5α** lab strain were also used, in order to discard possible detection of cross-reactivity antibodies.

**Table 1 - Peptides and proteins used for Lyme disease detection**

| Infection | Peptide/Protein |
|---|---|
| Acute | P41 |
| | OspC |
| | C2 19-mer |
| | C6 25-mer |
| Chronic | OspE1 |
| | OspE2 |
| | OspA |
| | DbpA |
| Autoimmune | LFA-1 |
| | Collagen I |
| | MBP |
| | Arp |
| Coinfections | *Babesia microti* |
| | *Ehrlichia N-t* |

**Table 2 - Positive Serum Samples**

| Immunoglobulin | IgG bands | IgM bands |
|---|---|---|
| Acute infection, IgG and IgM positive | P41, VlsE | OspC, p41 |
| IgG positive | OspC, p39, p41, p83, VlsE | Negative |
| Chronic infection, IgG positive | P58, OspC, p39, p41, p83, LBb, VlsE | Negative |
| IgG positive | P41, p83, Lba, VlsE | Negative |
| Chronic infection, IgG positive | P58, OspC, p39, p41, p83, LBb, VlsE | Negative |
| Chronic infection, IgG and IgM positive | P18, p20, OspC, p41, p83, VlsE | OspC |
| Chronic infection, IgG and IgM positive | P21, p39, p41, p83, VlsE | OspC, p41 |
| IgG positive bands and high Chlamydia pneumonia activity (IgA and LTT) | P58, p39, p41, p83, LBb, VlsE | Negative |
| IgG positive | P21, p39, p41, p83, LBb, VlsE | Negative |
| Chronic infection, IgG positive | P58, p39, p41, p83, VlsE | Negative |
| Chronic infection, IgG and IgM positive | P19, p58, OspC, p39, p41, p83, LBb, VlsE | OspC |
| Chronic infection, IgG and IgM positive | P21, OspC, p41, VlsE | OspC, p41 |
| Chronic infection, IgG positive | P39, p41, p83, LBb, VlsE | Negative |
| Chronic infection, IgG and IgM positive | OspC, p41, op83, VlsE | OspC, p41 |

Serum samples from patients confirmed with Lyme disease were tested by ELISA against the antigens previously described, in order to select the best immunogenic response antigens for the biochip.

Considering the results, all tested samples were IgG and/or IgM positive for at least one antigen, confirming the Lyme disease diagnosis obtained by commercial kits. These results showed that the inclusion of the pre-selected antigens grants a sensitivity of 100% to detect samples infected with Borrelia. However, not all the ELISA tested samples reacted with the "classical" antigens included in commercial kits (p41, OspC and VlsE), achieving lower sensitivity and specificity than expected (Table 3).

**Table 3 - Sensitivity and specificity of "classical antigens in ELISA test**

| Antigens | IgG sensitivity (%) | IgG specificity (%) | IgM sensitivity (%) | IgM specificity (%) |
|---|---|---|---|---|
| P41 | 21,4 | 21,4 | 14,3 | 0 |
| OspC | 35,7 | 28,6 | 50,0 | 50,0 |
| C2 19-mer | 42,8 | 42,8 | - | 100,0 |
| C6 25-mer | 57,1 | 57,1 | - | 100,0 |

Autoimmune conditions are thought to be responsible of long-term chronic manifestations of Lyme disease. Thus, in a serological assay the positive autoimmune antigens should be detected nearly together with chronic ones. In the results obtained with autoimmune antigens, all the IgG positive samples for LFA 1 were positive for the chronic markers OspA, OspEl-2, and/or DbpA, suggesting that this antigen is highly specific to detect Lyme disease patients in chronic stages with arthritic episodes. Specially, samples in which OspA and LFA 1 were both detected could correspond to patients at the beginning of these arthritic episodes, when OspA was supposed to cross-react with LFA 1. The same results were obtained with the MBP antigen in which the IgM positive serum sample was also positive for IgM OspA and DbpA. In the same way, Arp positive samples also coincided with the ones showing OspA and DbpA IgM response. Considering the novelty of this antigen and since more samples were detected with this protein when compared to IgM MBP results, the above mentioned negative sera were also used to better characterized the Arp serological sensitivity. Once included, the results confirmed the previous Arp positive samples obtained. Thus, rArp inclusion in the antigen bouquet of the present invention was considered due to the higher number of IgM positive samples obtained, when comparing with the other IgM autoimmunoreactive antigen (MBP). Regarding collagen, a well-known characterized antigen, all the positive IgG samples coincided with positive OspA, OspEl-2, and DbpA. In this case, when comparing this classical antigen with the other autoimmune antigen IgG positive (LFA 1), collagen seemed to be less specific.

Regarding coinfections, some samples seemed to be infected with *Babesia* and *Ehrlichia,* respectively, representing probable cases of babesiosis and ehrlichiosis. These positive results reinforce the fact that these antigens should be included in a Lyme diagnosis test, in the antigen bouquet of the present invention, in order to detect possible coinfections by other spirochete pathogens.
When analyzing cyst results, a vigorous IgG response was surprisingly observed, suggesting that the inclusion of this antigen in a new test could represent a real and novelty improvement in the sensitivity and specificity of Lyme disease diagnosis.
Finally, the fact that all the samples were negative for E. *coli* lysates indicated that any potential cross-reactivity of all the recombinant proteins synthesized in this cellular system (including rDbpA and rArp) can be discarded.

A preferred embodiment of the antigen bouquet of the present invention is Figure 3.

### 2 - Biochip

The biochip of the present invention (Figure 1) comprises the bouquet of antigens (described above and with a preferred embodiment in Figure 3) in microfluidic chambers, and was built as follows:
a) all functions are integrated, preferably without external connections (input reservoirs, valves, mixers, area detection and fluid pumps);
b) the biochip has the desired properties of smoothness and moisture content of the wall structure of channels of fluidics and a high ratio of appearance to allow strong capillary forces.
c) the biochip has pumps, preferably cappillary pumps, that allow the transport of the sample and reagents up to the detection area. This structure has a flow rate in the order of about 0,4 µL/min, allowing a total assay time under 20 minutes, and is capable of pumping the entire volume of the test sample through the detection chamber.
d) has a camera detection, in which the immunoglobulins of human serum, against the antigens of Borrelia, are captured by the immobilized antigen, with a capacity of more than 1000 immobilization of protein molecules. The cameras are designed to be compatible with a chemo-luminescence specific Reader and include the structures required for routing of wave lengths.
e) the approach to the production of a new low cost Biochip, highly reproducible and reliable.
f) Procedures for immobilisation of the antigen in the camera detection area and the conjugation of quantum Dots (qDots) with secondary antibodies against biological samples, preferably fluid samples and more preferably serum or cerebrospinal fluid, to be incorporated as reagents for stocking inside the biochip. The quality of the coating on the surface of the camera, and the qDots were evaluated using state -of- art techniques.
g) integration of low cost pumps and valves in the biochip not requiring external actuators.
h) open architecture design of the biochip, which can easily be adapted to other applications and diseases.

### 3 - Optical reader

The main objective of this deliverable is to present the development of a complete user-friendly automated reader specifically adapted for highly sensitive fluorescent read-out technology with Quantum Dots (QD) fluorescent markers performed in the biochip of the present invention. The Quantum Dots are a marker added to the system in order to measure how biochemical markers interact with patient serum or other biological, preferably fluid, samples.

This research also takes into account the cost over efficiency ratio. The aim was to use techniques that are possible to industrialize at a reasonable production cost for small to medium volumes.
The biochip optical reader of the present invention is for highly sensitive fluorescent read-out technology comprising a fluorescence detector containing an inner structure comprising a light source, preferably LED (16), a camera (17), a lens tube (18), Kohler illumination system (19), motorized translation stage for auto-focus (20), a mechanical frame (21), and a fixed objective positioner (22), and such fluorescence detector containing an intermediate structure comprising light shields (23), a housing frame (24), a plug panel (25), a stiffener (26), a computer compartment, an outer structure comprising the main casing (27), a touch screen (28), ventilation holes (29) and a trap door (30) wherein the fluorescence detector is specifically designed to use quantum dot fluorescent markers and is specifically adapted to be optically coupled and aligned with a biochip insertion and removal system (31) through a clamping system (32) and an auto-focus system (20), having a detection field of view up to 1 mm in length, preferably 480 µm, and up to 1 mm in width, preferably 480 µm, such detection field of view comprising the biochip field of view (A4) with about 65 µm tolerance in both X and Y-axis for positioning accuracy. The clamping system (32) comprises three contact points of reference with mechanical stoppers (33) with adjustable position and two pushers (34) which push the biochip against these stoppers wherein in addition to the X, Y clamps, two elastic clamps hold the biochip in position along the Z-axis and the return springs (35) pull back the pushers to allow the insertion and removal of the biochip. The insertion and removal system (31) consists of a drawer system comprising a piece of magnetic stainless steel material and a guiding mechanism with two guideway chariots (36) per rail, where in the closed position the drawer is blocked by an electromagnet (37) wherein the contact between the drawer and the electromagnet ensures a perfect X-axis alignment between the detector and the biochip, keeping the detector isolated from light, and in the opened position the drawer is blocked with two permanent magnets (38) to maintain it in position during the biochip loading.

### 4 - Method of the present invention

The Biochip is loaded with at least 16 specific antigens for antibodies developed for Lyme disease. The biological sample, preferably serum or cerebrospinal fluid, supposedly infected is collected and loaded into the chambers of biochip, waits a few seconds so that the sample flows inside the biochip. Then, the excess of sample is removed and a mixture of reagents and chemical buffers is loaded into the chamber, a process that is repeated twice. After this procedure, the biochip is inserted into the specifically adapted optical reader to be analysed with the help of an easy to use software, the algorithm of wish was developed in the present invention (Figure 7).

The antibodies for secondary linkage (known as "secondary antibodies"), who recognize the IgM and IgG antibodies attached to the antigens, are conjugated with Quantum Dots. When an antibody in the sample binds to an antigen and afterwards with a secondary antibody, the following connection is expected to occur: antigen - sample antibody - secondary antibody - Quantum dot. Thus, when this connection occurs in the biochip of the present invention, the Biochip Reader of the present invention will excite these Quantum Dots which in turn transmit fluorescence, reading this signal as a positive indication of the presence of infection in the sample and further interpretation parameters apply regarding the stage (early, acute or chronic) and type (autoimmune or coinfection) for the case of Lyme disease.

### BRIEF DESCRIPTION OF THE DRAWINGS

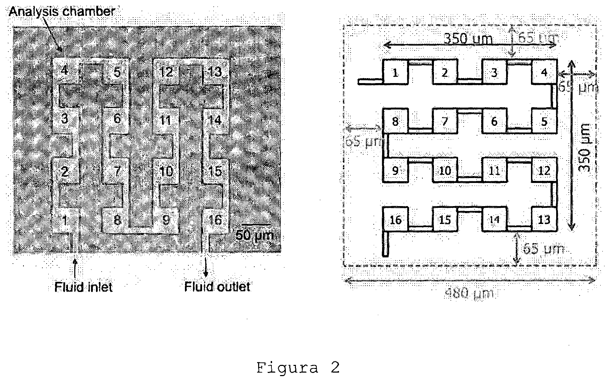

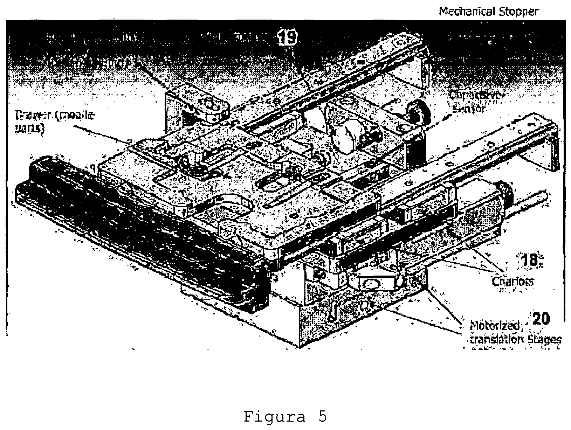

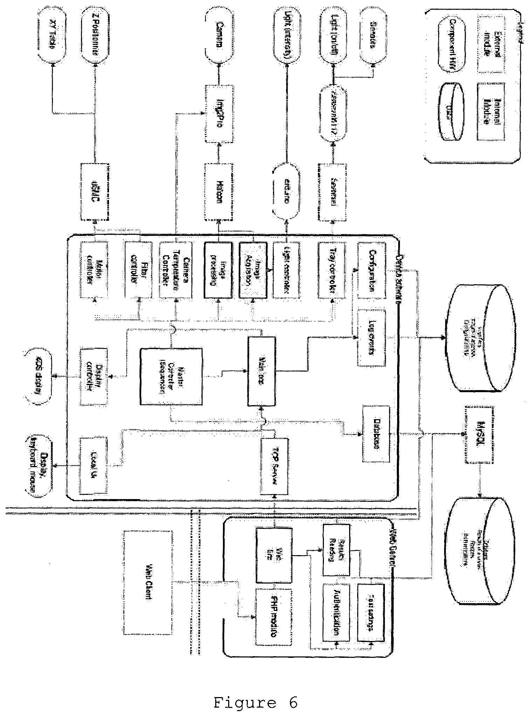

Figure 1 - Design and configuration of the biochip:
   1 - Reservoir 1 (sample)
   2 - Reservoir 2 (PBS1 and PBS2 washings)
   3 - Reservoir 3 (Reagent)
   4 - Working area (field of view and capillary valves)
   5 - Pump 1
   6 - Pump 2
   7 - Microfluidic channel 1
   8 - Microfluidic channel 2
   9 - Microfluidic channel 3
   10 - Microfluidic channel 4
   11 - Microfluidic channel 5
   12 - Microfluidic channel 6
   13 - Microfluidic channel 7
   14 - Microfluidic channel 8
   15 - Microfluidic channel 9
Figure 2 - Optical micrograph of the field of view (4) comprising analysis chambers with up to 50 µm length, up to 50 µm width and up to 15 µm deep.
Figure 3 - Antigen bouquet for differential detection of Lyme disease.

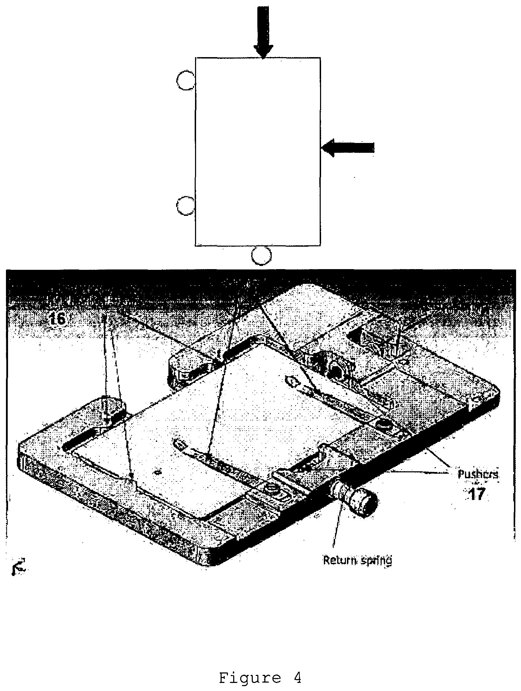

Figure 4 -Clamping system of the biochip optical reader
   16 - mechanical stoppers
   17 - two pushers
Figure 5 - Insertion and removal system of the biochip optical reader
   18 - two guideway chariots
   19 - electromagnet
   20 - two permanent magnets
Figure 6 - Software algorithm architecture as implemented on the biochip optical reader.

### DETAILED DESCRIPTION OF THE INVENTION

### 1. Bouquet of 16 antigens

An innovative set of antigens was designed, which we call a new *bouquet* of 16 specific antigens, to be used in whole or in part for the detection of Lyme disease, they being described as: IgG and IgM, both used as positive control for the immune reaction; Cell Lysates of *E. Coli,* used as negative control for infection of *Borrelia burgdorferi f.l.;* OspC (Outer Surface Protein C), C2 19-mer (DAASVNGIAKGIKGIVDAA) and C6 25-mer (MKKDDQIAAAMVLRGMAKDGQFALK). All together, these are antigens of acute infection caused by *Borrelia burgdorferi f.l.;*
OspEl/2 (Outer Surface Protein ANDl/2) OspA (Outer Surface Protein A) and DbpA (Decorin binding protein A). Together, these are antigens of chronic infection caused by *Borrelia burgdorferi f.l.;*
LFAl (Lymphocyte Function Antigen-1) and Arp (Arthritis-related proteins GST-like self-antigen), that are autoimmune antigens of infection caused by *Borrelia burgdoferi;*
Peptides of *Babesia microti* (with the sequence IVEFNAIFSNIDLNNSSTVKNEIIK) and *Ehrlichia* (with the sequence SAVSNRKLPLGGVLMALVAAVAPIHSALLA), for detecting the occurrence of co-infections;
*Borrelia burgdorferi* B31 spirochete, *B. burgdorferi* B31 cysts, *Borrelia garinii* and *Borrelia azfelii,* used as cell lysates as positive control for infection by *Borrelia* and for their nearest related species.

In this bouquet, 14 of antigens had already been discovered and 2 antigens were discovered and produced specifically for this *bouquet* for detection of Lyme disease, being here identified as the antigens **DbpA** and **Arp.**

### 1.1 Cloning of the DNA coding for antigen Decorin binding protein A (DbpA)

To express the antigen DbpA (rDbpA), we selected the plasmid pBAD/His for inducible expression in *E. Coli.* The protein will be expressed as a fusion protein with a 6-His-Tag in position N-terminal. The 6-His-tag is small, not immunogenic and will allow the purification through connection matrices of ionized metal.

Initially the expression plasmid was amplified and purified (Midiprep QIAfilter Plasmid Purification Kit # 12243). Subsequently, 2 µg of the purified plasmid and the entire amplicon DbpA were digested with the restriction enzymes *HindIII Xhol* during the night at 37°C, following the conditions of Table 4 described below:

**Table 4**

| **Digestion with XhoI and HindIII** | **Plasmid (µl)** | **DbpA (µl)** |
|---|---|---|
| Deionized Water and free from nucleases | 25,5 | 35 |
| DNA template | 15 | 50 |
| NEB 2 buffer (10x) | 5 | 10 |
| BSA (100x) | 0,5 | 1 |
| XhoI (20 u/ul) | 2 | 2 |
| HindIII (20 u/ul) | 2 | 2 |
| **Final Volume** | **50** | **100** |

The plasmid and amplicon from DbpA protein were seen on a 0,8% agarose gel (330 V for 70 minutes) and purified with QiAQuick Gel Extraction. The cleavaged DbpA was then connected to the digested plasmid, in a ratio of 1:3, for 3 hours at room temperature, and 5 µL transformed into cells *E. coli* NEB 5-alpha (25 µL). The mixture of transformation (200 µL) was placed on plates *Luria-Bertani* (LB), containing these plates 50 µg of ampicillin per mL, and grown during the night at 37 °C.

**Table 5**

| **Reagents used in the reaction of ligation** | **Ratio 1:3 (µL)** | **Without the plasmid insertion (µl)** | **Without plasmid insertion and DNA ligase (µl)** |
|---|---|---|---|
| Deionized water and free from nucleases | 5 | 7 | 8 |
| T4 Ligase Buffer (10x) | 2 | 2 | 2 |
| Plasmid DNA (∼10 ng/µl) | 10 | 10 | 10 |
| Plasmid insertion (∼25 ng/µl) | 2 | - | - |
| T4 DNA ligase (3U/µl, Promega, #M1801) | 1 | 1 | - |
| Final Volume | 20 | 20 | 20 |

Several positive colonies are amplified in 2 mL of LB (165 µg of ampicillin/mL), purified (QIAfilter Plasmid Miniprep Purification Kit # 37104), digested with *Xhol*/*HindIII* at 37°C for 2 hours, run in agarose gel (1%), to confirm the presence of the gene DbpA. 5 Minipreps are obtained and digested confirmed as positive, of which 2 are selected for sequencing. After confirmed that the sequence exactly matches the gene DbpA (included the His-tag), 200 µl of one of the positive cultures of minipreps processed with TOP10, is grown in 200 mL of LB (100 µg of ampicillin/mL) at 37 °C during the night. The selection of TOP10 cell is because it is a strain of recA/endA, capable of transporting L- arabinose, but not capable of its metabolization. The DNA of DbpA construct of these 200 mL of culture is purified with Qiafilter Plasmid Midi kit (Qiagen #12243), transformed (2,8 µl, ∼100 ng) in cells TOP10 (15 µl), and grown in plates of LB (100 µg/mL ampicillin) at 37°C during the night. Finally, one of the colonies was again replaced to generate stocks in glycerin and for expressing rDbpA.

### 1.2. Cloning of antigen Arthritis-related proteins GST-like self-antigen (Arp)

For expression of the recombinant protein Arp (rArp), the same plasmid used in the expression of the DbpA, plasmid pBAD/His (Invitrogen) is used to express the protein 6-His- tag. The recombinant plasmid is amplified and purified as described in the previous section to the DbpA, but instead of *HindIII, EcoRI* was used. 1 µg of plasmid and the entire gene amplified and purified Arp were digested with *Xhol* and EcoRI for 3 hours at 37 °C, following the conditions of the following table:

**Table 6**

| **Reagents for digestion with XhoI and EcoRI** | **Plasmid (µl)** | **Arp (µl)** |
|---|---|---|
| Deionized Water and free from nucleases | 40 | 11,5 |
| DNA template | 1,5 | 30 |
| NEB 4 buffer (10x) | 5 | 5 |
| BSA (100x) | 0,5 | 0,5 |
| XhoI (20 u/ul) | 1,5 | 1,5 |
| EcoRI (20 u/u) | 1,5 | 1,5 |
| **Final Volume** | **50** | **50** |

The plasmid and protein were separated on a 0,8% agarose gel (330 V for 70 minutes) and purified with QiAQuick Gel Extraction. The cleavaged Arp was then connected to the plasmid of digestion, in a ratio of 1:3, during 75 minutes at ambient temperature, and 5 µL transformed into cells *E. coli* NEB 5-alpha (25 µL) . The mixture of transformation (200 µL) was placed on plates *Luria-Bertani* (LB), containing these plates 50 µg of ampicillin per mL, and grow during the night at 37 °C.

**Table 7**

| **Reagents for reaction of connection** | **Ratio 1:3 (µL)** | **Without the plasmid insertion (µL)** | **Without plasmid insertion and DNA ligase (µL)** |
|---|---|---|---|
| Deionized Water and free from nucleases | 12,7 | 14,5 | 15,5 |
| T4 Ligase Buffer (10x) | 2 | 2 | 2 |
| Plasmid DNA (∼10 ng/µl) | 2,5 | 2,5 | 2,5 |
| Plasmid insertion (∼25 ng/µl) | 1,8 | - | - |
| T4 DNA ligase (3U/µl, Promega, #M1801) | 1 | 1 | - |
| **Final Volume** | **20** | **20** | **20** |

Several colonies positive were amplified in 2 mL of LB (165 µg of ampicillin/mL), purified (QIAfilter Plasmid Miniprep Purification Kit # 37104), digested with *Xhol*/*EcoRI* at 37°C during the night, and separated in agarose gel (1%), to confirm the presence of the gene Arp. Two colonies of positive minipreps were selected for sequencing. After confirmed that the sequence exactly matches the gene Arp (included the His-tag), 200 µL of one of the positive cultures of minipreps processed with TOP10, grow in 200 mL of LB (100 µg of ampicillin/mL) at 37 °C during the night. From this 200 mL of culture, the DNA of Arp construct was purified with Qiafilter Plasmid Midi kit (Qiagen #12243, transformed (1 µL, ∼100 ng) in cells TOP10 (15 µL), and grows in plaques of LB (100 µg/mL ampicillin) at 37 °C during the night. Finally, one of the colonies was again replicated to generate stocks in glycerin and for expressing rArpn.

### 2. Interpretation of results the Biochip

A sample of the patient, which is possibly infected with the Lyme disease is initially collected. This sample can be a fluid (urine, whole blood, serum, plasma, cerebrospinal fluid, sweat, saliva) or tissue lysates (of different tissues). Add about 3 µL of the filtered and diluted solution (1/200) of the sample in 0,1% Tween 20/PBS and leave to the appropriate entry of the Biochip during 9 minutes. Then remove the excess of sample and wash the respective entry with about 3 µL of a filtered solution of 0,1% Tween 20/PBS. Goes back to remove the excess of the previous solution and adds about 3 µL of a solution of 1/10 and 1/100 of qDot525 and qDot625 functionalized secondary antibodies and leave it at that entry during 9 minutes. Again, removes the excess of the previous solution and goes back to perform a washing operation with about 3 µL of a solution of 0,1% Tween 20/PBS. After this procedure is performed, the biochip is ready to be read in the optical biochip reader of the present invention.

The qDots listed above are fundamental, because they are revealing agents that will allow the identification of the linkage between the first agent of connection of the reaction (one of 16 antigens fixed in Biochip) with the analyte (sample) and then with the second agent of connection to the trapped antibody (IgG or IgM). If there is a linkage between the first agent of connection of, the analyte, and then with the second agent of ligation of the reaction, when the biochip is scanned in the optical reader of the present invention, fluorescence will be emitted, indicating that the test was positive for Lyme disease and the patient is infected with the bacterium Borrelia. If a connection does not occur, this means that the sample of the patient does not have antibodies against the bacterium *Borrelia burgdorferi f.l.* and the patient is not infected with Lyme disease in any extent.

### 3. Quantum Dots, revealing agents

QuantumDots (qDots) are a distinct group of fluorescent compounds that differ from conventional methods in that they have unique spectral characteristics. Their cores of heavy metals are responsible by these original spectral characteristics and are composed of potentiometer material: selene to cadmium (CdSe) or indium phosphide And gallium (InGaP). The cores usually possess technological properties of coating that allows the combination with various biomolecules, including antibodies, proteins and peptides. This coating is also a polymer which provides a surface soluble in water that can modify the combination with other biomolecules such as antibodies and proteins. These qDots are adjustable in size (2-50 nm), usually of 10-20 nm, have spherical shape, that allows qDots to be adjusted to a specific wavelength emission. The qDots are intrinsically bright, have very narrow emission spectra, have photo-stability, are easy to use, and can be used in a multitude of applications. The use of these Dots in biomedical applications is just emerging and gaining popularity. Due to their spectral characteristics the qDots have fundamental high performance in a variety of applications, such as flow cytometry, immuno-histochemistry or in our case, as molecules of detection for human sera and other biological samples.

In the case of this invention, qDots 525 and 625 were chosen particularly for the case of the antigen bouquet used for detecting Lyme disease. The number mentioned after qDots corresponds to the emission peak of the said qDot, in our case the qDots chosen for this invention will have peaks of emission at 525 nm (qDots 525) and 625 nm (qDots 625). Both qDots will be combined with secondary antibodies that are anti-human IgG or anti-human IgM forming the second complex "Legend-Qdot". This complex will recognise and connect to the first complex ligand-analyte (human serum), in case that serum comprises antibodies against *Borrelia burgdorferi f.l.* If the Ligand immobilized (antigen) - analyte (serum antibodies) - Second Ligand (secondary antibody) - qDot complex is present when the Biochip is placed on the Reader of chemo-luminescence, the qDots will be excited to the wavelengths referred above, and will transmit fluorescence, accusing a positive diagnosis for Lyme disease. Preferably, for Lyme disease, IgG will be revealed and quantified by qDot 525 and IgM will be revealed and quantified by qDot 625, in order to differentiate not only the different stages (early, acute and chronic) or absence of the disease but also to detect antibodies associated to autoimmunity related to Lyme disease and coinfection between *B. burgdorferi* and other antigens.

## Claims

1. System for detecting and monitoring the presence of antibodies associated to different stages of Lyme disease, present in biological samples, comprising
- an antigen bouquet
- a biochip
- a biochip optical reader; and
**characterized by the fact that**
the antibodies present in the biological samples are detected specifically for acute, chronic, autoimmunity and coinfection related to Lyme disease, the antigen bouquet comprising sixteen antigens, in particular, IgG and IgM, cell lysates of E. coli, OspC, OspA, DbpA and Arp, OspE1/2, LFA1, Arp, peptides of Babesia microti and Ehrlichia, Borrelia burgdorferi B31 spirochete, Borrelia burgdorferi B31 cysts, Borrelia garinii and Borrelia azfelii;
and in that
said biochip comprises a first reservoir (1) suitable for containing biological samples, a second reservoir (2) for the washing buffer, a third reservoir (3) for quantum dots, a working area and field of view (4) comprising valves, reaction chambers and analysis chambers wherein the reaction and analysis chambers bear antigen coatings, with at least a first pump (5), a second pump (6) and nine microfluidic channels (7, 8, 9, 10, 11, 12, 13, 14 and 15).

2. System according to claim 1 **characterized by the fact that** the biological samples are serum or cerebrospinal fluid.

3. System according to claim 1 **characterized by the fact that** the washing buffer is 0,1% Tween 20/PBS solution.

4. System according to claim 1 **characterized by the fact that** the quantum dots fluoresce between 400 and 900nm, preferably between 500 and 700 nm, and more preferably at 525 nm and 625nm, and are conjugated to anti-human IgG or anti-human IgM antibodies.

5. System according to previous claims **characterized by the fact that** the biochip working area and field of view (42) measures up to 1 mm in length and up to 1 mm in width, preferably 350 µm in width, and comprises at least sixteen reaction and analysis chambers.

6. System according to previous claims **characterized by the fact that** each reaction and analysis chamber comprised within the working area and field of view (42) has up to 50 µm in length, up to 50 µm in width and is up to 15 µm deep.

7. System according to previous claims **characterized by the fact that** the biochip comprises capillary fluids pumps (43) and (44) with a flow rate of at least 0,2 pL/minute, preferably with a flow rate of 0,4 pL/minute.

8. System according to previous claims **characterized by the fact that** the biochip optical reader comprises a fluorescence detector containing an inner structure comprising a light source, preferably LED, a camera, a lens tube, Köhler illumination system, an auto-focus system, a mechanical frame, and a fixed objective positioner.

9. System according to claim 8 **characterized by the fact that** the biochip optical reader further contains an intermediate structure comprising light shields, a housing frame, a plug panel, a stiffener, a computer compartment and an outer structure comprising the main casing, a touch screen, ventilation holes and a trap door, where the fluorescence detector is specifically designed to use quantum dot fluorescent markers and is specifically adapted to be optically coupled and aligned with a biochip insertion and removal system through a clamping system and the auto-focus system has a detection field of view up to 1 mm in length, preferably 480 µm, and up to 1 mm in width, preferably 480 µm, and that the detection field of view comprises the biochip field of view (4) with about 65 µm tolerance in both X and Y-axis for positioning accuracy.

10. System according to claim 9 **characterized by the fact that** the biochip optical reader clamping system comprises three contact points of reference with mechanical stoppers (16) with adjustable position and two pushers (34) which push the biochip against these stoppers wherein in addition to the X, Y clamps, two elastic clamps hold the biochip in position along the Z-axis and the return springs (17) pull back the pushers to allow the insertion and removal of the biochip.

11. System according to claim 9 **characterized by the fact that** the biochip optical reader insertion and removal system consists of a drawer system comprising a piece of magnetic stainless steel material and a guiding mechanism with two guideway chariots (18) per rail, where in the closed position the drawer is blocked by an electromagnet (19) wherein the contact between the drawer and the electromagnet ensures a perfect X-axis alignment between the detector and the biochip, keeping the detector isolated from light, and in the opened position the drawer is blocked with two permanent magnets (20) to maintain it in position during the biochip loading.

12. Method of using the system for detecting and monitoring the presence of antibodies associated to Lyme disease in biological samples according to claims 1 to 11 **characterized by** the following steps:
a) execution of an algorithm in the optical reader;
b) addition of 3 µL of a filtered 1:200 dilution of the biological sample in 0,1% Tween 20/PBS for up to 9 minutes to the respective chamber;
c) a washing operation with 3 µL of filtered 0,1% Tween 20/PBS;
d) addition of 3 µL of a 1:10 dilution of quantum dot 525 and a 1:100 dilution of quantum dot 625 for up to 9 minutes;
e) a second washing operation with 3 µL of filtered 0,1% Tween 20/PBS;
f) insertion of the biochip in the optical reader's drawer;
g) entering information available from the web interface; and
h) pushing the start test button,
wherein the device places the biochip in an auto-focus system for up to 2 minutes, optionally waits for the end of migration, performs image analysis for up to 2 minutes, stores and displays results on the web page in up to 5 seconds.

13. Method according to claim 12 **characterized by the fact that** the biological samples are preferably serum or cerebrospinal fluid.

14. Method of using the system of claims 1-11, according to claims 12 to 13, **characterized by the fact that** the biochip is inserted and removed through a clamping system which comprises three contact points of reference with mechanical stoppers (16) with adjustable position and two pushers (34), which push the biochip against these stoppers wherein in addition to the X, Y clamps, two elastic clamps hold the biochip in position along the Z-axis and the return springs (17) pull back the pushers to allow the insertion and removal of the biochip.

15. Method of using the system of claims 1-11, according to claims 12-14, **characterised by the fact that** the clamping system is part of a drawer system comprising a piece of magnetic stainless steel material and a guiding mechanism with two guideway chariots (18) per rail, wherein the closed position of the drawer is blocked by an electromagnet (19), and the contact between the drawer and the electromagnet ensures a perfect X-axis alignment between the fluorescence detector and the biochip, keeping the fluorescence detector isolated from light, and in the opened position the drawer is blocked with two permanent magnets (20) to maintain it in position during the biochip loading.

16. Method of using the system of claims 1-11, according to claims 12-15, **characterised by the fact that** the auto-focus system comprises a Z axis translation stage with a ball bearing guided table motorized by a brushed DC servo motor and operates through three steps:
a) scans a large range of distances between the biochip and the objective, in the order of 1mm, with large movement steps in the order of 50µm, between each image capture, wherein the movement of Z-axis is continuous and pictures are taken on-the-fly and the distance, for which the image is the sharpest, is recorded;
b) scans accurately, with small movements in the order of a few microns, preferably between 5 and 50µm, the distance range determined by the first step in order to find the in-focus distance, Z-axis moves and then a picture is acquired when Z-axis is stopped and, as soon as the whole range of second step is scanned, the Z-axis moves back to the position corresponding to the sharpest image; and
c) moves down the Z-axis by a distance corresponding to the depth of microfluidic channels of the biochip, preferably between 15 and 20µm and then moves down the Z-axis by up to 10µm to compensate the optical difference between bright field filter and fluorescence filters.

17. Method of using the system of claims 1-11, according to claims 12-16, **characterised by the fact that** the algorithm extracts the exact location of the different reaction chambers within the sharp image collected in claim 16, performs image analysis and produces a report, comprising:
a) scaling down to a lower resolution image;
b) transformation of image from temporal domain to frequential domain, using Fourier transform;
c) correlation of the frequential image with itself through auto-correlation;
d) inverting Fourier transform of the auto-correlated image;
e) mapping of the "inverse Fourier image" into pixel domain;
f) computation of the mean and standard deviation of pixel intensities of the original image of lower resolution;
g) computation of the correlation factor between original image, such as mean and standard deviation, and pixels of the "inverse Fourier image";
h) computation of the score based on the minimum of correlation factor of previous step;
i) finding the field of view, its position and orientation through pattern matching, wherein patterns are created by drawing about 16 regions of interest aligned with the pattern, being these about 16 regions correspondent to the areas where data are used to provide diagnosis test results; and
j) pattern matching algorithm is used with the pattern stored from the previous step;
wherein new image analysis algorithm works in 4 consecutive steps, namely determination of best camera exposure time, determination of residues and the removal thereof, determination of background fluorescence and analysis of reaction chambers without residues and background fluorescence.

18. Method of using the system of claims 1-11, according to claims 12-17, **characterised by the fact that** it comprises the execution of a customized algorithm in the optical reader and:
a) addition of 3µL of a filtered 1:200 dilution of the biological sample in 0,1% Tween 20/PBS for up to 9 minutes to the respective chamber;
b) a washing operation with 3µL of filtered 0,1% Tween 20/PBS;
c) addition of 3µL of a 1:10 dilution of quantum dot 525 and a 1:100 dilution of quantum dot 625 for up to 9 minutes;
d) a second washing operation with 3µL of filtered 0,1% Tween 20/PBS;
e) insertion of the biochip in the optical reader's drawer;
f) entering information available from a web interface; and
g) pushing the start test button;
wherein the device places the biochip in an auto-focus system for up to 2 minutes, optionally waits for the end of migration, performs image analysis for up to 2 minutes, stores and displays results on the web page in up to 5 seconds.

## Patentansprüche

1. System zum Nachweis und zur Überwachung des Vorhandenseins von Antikörpern, die mit verschiedenen Stadien der Lyme-Krankheit assoziiert sind, die in biologischen Proben vorhanden sind, umfassend
- ein Antigen-Bouquet
- einen Biochip
- einen optischen Biochip-Leser; und
**dadurch gekennzeichnet, dass**
die in den biologischen Proben vorhandenen Antikörper spezifisch für akute Infektion, chronische Infektion, Autoimmunität und Co-Infektion im Zusammenhang mit der Lyme-Krankheit nachgewiesen werden, wobei das Antigen-Bouquet sechzehn Antigene, insbesondere IgG und IgM, Zelllysate von E. Coli, OspC, OspA, DbpA und Arp, OspE1/2, LFA1, Arp, Peptide von Babesia microti und Ehrlichia, Spirochäte Borrelia burgdorferi B31, Borrelia burgdorferi B31-Zysten, Borrelia garinii und Borrelia azfelii, umfasst;
und dass
der Biochip einen ersten Behälter (1), der geeignet ist, biologische Proben aufzunehmen, einen zweiten Behälter (2) für den Waschpuffer, einen dritten Behälter (3) für Quantenpunkte, einen Arbeitsbereich und ein Sichtfeld (4), umfassend Ventile, Reaktionskammern und Analysekammern, wobei die Reaktions- und Analysekammern Antigenbeschichtungen tragen, umfasst, mit mindestens einer ersten Pumpe (5), einer zweiten Pumpe (6) und neun Mikrofluidikkanälen (7, 8, 9, 10, 11, 12, 13, 14 und 15).

2. System nach Anspruch 1, **dadurch gekennzeichnet, dass** die biologischen Proben Serum oder Liquor sind.

3. System nach Anspruch 1, **dadurch gekennzeichnet, dass** der Waschpuffer 0,1 % Tween 20/PBS-Lösung ist.

4. System nach Anspruch 1, **dadurch gekennzeichnet, dass** die Quantenpunkte zwischen 400 und 900 nm, vorzugsweise zwischen 500 und 700 nm und insbesondere bevorzugt bei 525 nm und 625 nm fluoreszieren und mit Anti-Human-IgG- oder Anti-Human-IgM-Antikörpern konjugiert sind.

5. System nach den vorhergehenden Ansprüchen, **dadurch gekennzeichnet, dass** der Arbeitsbereich und das Sichtfeld (42) des Biochips bis zu 1 mm lang und bis zu 1 mm breit, vorzugsweise 350 µm breit sind und mindestens sechzehn Reaktions- und Analysekammern umfassen.

6. System nach den vorhergehenden Ansprüchen, **dadurch gekennzeichnet, dass** jede Reaktions- und Analysekammer, die sich innerhalb des Arbeitsbereichs und des Sichtfeldes (42) befindet, bis zu 50 µm Länge, bis zu 50 µm Breite und bis zu 15 µm Tiefe aufweist.

7. System nach den vorhergehenden Ansprüchen, **dadurch gekennzeichnet, dass** der Biochip Kapillarfluidpumpen (43) und (44) mit einer Fördermenge von mindestens 0,2 µL/Minute, vorzugsweise mit einer Fördermenge von 0,4 µL/Minute, umfasst.

8. System nach den vorhergehenden Ansprüchen, **dadurch gekennzeichnet, dass** der optische Biochip-Leser einen Fluoreszenzdetektor umfasst, der eine innere Struktur enthält, die eine Lichtquelle, vorzugsweise LED, eine Kamera, eine Linsenröhre, ein Köhler-Beleuchtungssystem, ein Autofokussystem, einen mechanischen Rahmen und einen festen Objektivpositionierer umfasst.

9. System nach Anspruch 8, **dadurch gekennzeichnet, dass** der optische Biochip-Leser ferner eine Zwischenstruktur enthält, die Lichtschirme, einen Gehäuserahmen, eine Steckerplatte, eine Versteifung, ein Computerfach und eine Außenstruktur, umfassend das Hauptgehäuse, einen Touchscreen, Lüftungslöcher und eine Falltür, umfasst, wobei der Fluoreszenzdetektor gezielt für die Verwendung von Quantenpunkt-Fluoreszenzmarkern ausgelegt ist und gezielt für die optische Kopplung und Ausrichtung mit einem Biochip-Einlege- und Entfernungssystem durch ein Klemmsystem angepasst ist und das Autofokussystem ein Detektionssichtfeld von bis zu 1 mm, vorzugsweise 480 µm Länge, und bis zu 1 mm, vorzugsweise 480 µm Breite aufweist und dass das Detektionssichtfeld das Biochip-Sichtfeld (4) mit einer Toleranz von etwa 65 µm sowohl in X- als auch in Y-Achse für die Positionierungsgenauigkeit umfasst.

10. System nach Anspruch 9, **dadurch gekennzeichnet, dass** das Klemmsystem des optischen Biochip-Lesers drei Referenzkontaktpunkte mit mechanischen Stoppern (16) mit einstellbarer Position und zwei Drückern (34) umfasst, die den Biochip gegen diese Stopper drücken, wobei zusätzlich zu den X-, Y-Klemmen zwei federnde Klemmen den Biochip in Position entlang der Z-Achse halten und die Rückstellfedern (17) die Drücker zurückziehen, um das Einlegen und Entfernen des Biochips zu ermöglichen.

11. System nach Anspruch 9, **dadurch gekennzeichnet, dass** das Einlege- und Entfernungssystem des optischen Biochip-Lesers aus einem Schubladensystem besteht, das ein Stück magnetisches Edelstahlmaterial und einen Führungsmechanismus mit zwei Führungswagen (18) pro Schiene umfasst, wobei in der geschlossenen Position die Schublade durch einen Elektromagneten (19) blockiert wird, wobei der Kontakt zwischen der Schublade und dem Elektromagneten eine perfekte X-Achsenausrichtung zwischen dem Detektor und dem Biochip gewährleistet, wobei der Detektor vom Licht isoliert gehalten wird, und in der geöffneten Position die Schublade mit zwei Permanentmagneten (20) blockiert wird, um sie während der Ladung des Biochips in Position zu halten.

12. Verfahren zur Verwendung des Systems zum Nachweis und zur Überwachung des Vorhandenseins von Antikörpern, die mit der Lyme-Krankheit assoziiert sind, in biologischen Proben, nach Anspruch 1 bis 11, **gekennzeichnet durch** folgende Schritte:
a) Ausführen eines Algorithmus im optischen Leser;
b) Zugabe von 3 µL einer gefilterten 1:200 Verdünnung der biologischen Probe in 0,1 % Tween 20/PBS für bis zu 9 Minuten in die jeweilige Kammer;
c) einen Waschvorgang mit 3 µL gefiltertem 0,1 % Tween 20/PBS;
d) Zugabe von 3 µL einer 1:10-Verdünnung von Quantenpunkt 525 und einer 1:100-Verdünnung von Quantenpunkt 625 für bis zu 9 Minuten;
e) einen zweiten Waschvorgang mit 3 µL gefiltertem 0,1 % Tween 20/PBS;
f) Einsetzen des Biochips in die Schublade des optischen Lesers;
g) Eingabe von Informationen, die über die Webschnittstelle verfügbar sind; und
h) Drücken der Starttesttaste, wobei die Vorrichtung den Biochip für bis zu 2 Minuten in ein Autofokussystem stellt, optional auf das Ende der Migration wartet, eine Bildanalyse für bis zu 2 Minuten durchführt, Ergebnisse speichert und auf der Webseite in bis zu 5 Sekunden anzeigt.

13. Verfahren nach Anspruch 12, **dadurch gekennzeichnet, dass** die biologischen Proben vorzugsweise Serum oder Liquor sind.

14. Verfahren zur Verwendung des Systems der Ansprüche 1-11 nach Anspruch 12 bis 13, **dadurch gekennzeichnet, dass** der Biochip durch ein Klemmsystem eingelegt und entfernt wird, das drei Referenzkontaktpunkte mit mechanischen Stoppern (16) mit einstellbarer Position und zwei Drückern (34) umfasst, die den Biochip gegen diese Stopper drücken, wobei zusätzlich zu den X-, Y-Klemmen zwei federnde Klemmen den Biochip in Position entlang der Z-Achse halten und die Rückstellfedern (17) die Drücker zurückziehen, um das Einlegen und Entfernen des Biochips zu ermöglichen.

15. Verfahren zur Verwendung des Systems der Ansprüche 1-11 nach Anspruch 12-14, **dadurch gekennzeichnet, dass** das Klemmsystem Teil eines Schubladensystems ist, das ein Stück magnetisches Edelstahlmaterial und einen Führungsmechanismus mit zwei Führungswagen (18) pro Schiene umfasst, wobei die geschlossene Position der Schublade durch einen Elektromagneten (19) blockiert wird, wobei der Kontakt zwischen der Schublade und dem Elektromagneten eine perfekte X-Achsenausrichtung zwischen dem Fluoreszenzdetektor und dem Biochip gewährleistet, wobei der Fluoreszenzdetektor vom Licht isoliert gehalten wird, und in der geöffneten Position die Schublade mit zwei Permanentmagneten (20) blockiert wird, um sie während der Ladung des Biochips in Position zu halten.

16. Verfahren zur Verwendung des Systems der Ansprüche 1-11 nach Anspruch 12-15, **dadurch gekennzeichnet, dass** das Autofokussystem eine Z-Achse-Translationsplattform mit einem kugellagergeführtem, von einem gebürsteten Gleichstrom-Servomotor angetriebenen Tisch umfasst und in drei Schritten arbeitet:
a) einen großen Bereich von Abständen zwischen dem Biochip und dem Objektiv, in der Größenordnung von 1 mm, mit großen Bewegungsschritten in der Größenordnung von 50 µm, zwischen jeder Bildaufnahme abtastet, wobei die Bewegung der Z-Achse kontinuierlich ist und Bilder im Vorbeigehen aufgenommen werden und der Abstand, für den das Bild am schärfsten ist, aufgezeichnet wird;
b) mit kleinen Bewegungen in der Größenordnung von wenigen Mikrometern, vorzugsweise zwischen 5 und 50 µm, den durch den ersten Schritt bestimmten Abstandsbereich genau abtastet, um den in-Fokusabstand zu finden, sich die Z-Achse bewegt und dann ein Bild aufgenommen wird, wenn die Z-Achse gestoppt wird, und sobald der gesamte Bereich des zweiten Schrittes abgetastet wird, sich die Z-Achse zurück in die Position bewegt, die dem schärfsten Bild entspricht; und
c) sich nach unten auf der Z-Achse um einen Abstand, der der Tiefe der Mikrofluidikkanäle des Biochips entspricht, vorzugsweise zwischen 15 und 20 µm bewegt, und sich dann nach unten auf der Z-Achse um bis zu 10 µm bewegt, um den optischen Unterschied zwischen Hellfeldfilter und Fluoreszenzfilter auszugleichen.

17. Verfahren zur Verwendung des Systems der Ansprüche 1-11 nach Anspruch 12-16, **dadurch gekennzeichnet, dass** der Algorithmus die genaue Position der verschiedenen Reaktionskammern innerhalb des in Anspruch 16 gesammelten scharfen Bildes extrahiert, eine Bildanalyse durchführt und einen Bericht erstellt, umfassend:
a) Verkleinern auf ein Bild mit geringerer Auflösung;
b) Transformation des Bildes von der Zeitdomäne in die Frequenzdomäne unter Verwendung der Fourier-Transformation;
c) Korrelation des Frequenzbildes mit sich selbst durch Autokorrelation;
d) Invertieren der Fourier-Transformation des autokorrelierten Bildes;
e) Abbildung des "inversen Fourier-Bildes" in die Pixeldomäne;
f) Berechnung des Mittelwerts und der Standardabweichung der Pixelintensitäten des Originalbildes mit geringerer Auflösung;
g) Berechnung des Korrelationsfaktors zwischen dem Originalbild, wie Mittelwert und Standardabweichung, und den Pixeln des "inversen Fourier-Bildes";
h) Berechnung des Scores basierend auf dem Minimum des Korrelationsfaktors des vorherigen Schrittes;
i) Finden des Sichtfeldes, seiner Position und Orientation durch Musterabgleich, wobei Muster durch Zeichnen von etwa 16 Bereichen von Interesse erzeugt werden, die mit dem Muster ausgerichtet sind, wobei diese etwa 16 Bereiche den Bereichen entsprechen, in denen Daten verwendet werden, um Diagnosetestergebnisse bereitzustellen; und
j) ein Musterabgleichsalgorithmus mit dem im vorherigen Schritt gespeicherten Muster verwendet wird;
wobei der neue Bildanalysealgorithmus in 4 aufeinanderfolgenden Schritten arbeitet, nämlich Bestimmen der besten Kamerabelichtungszeit, Bestimmen von Rückständen und deren Entfernung, Bestimmen der Hintergrundfluoreszenz und Analysieren von Reaktionskammern ohne Rückstände und Hintergrundfluoreszenz.

18. Verfahren zur Verwendung des Systems der Ansprüche 1-11 nach Anspruch 12-17, **dadurch gekennzeichnet, dass** es die Ausführung eines individualisierten Algorithmus in dem optischen Leser umfasst und:
a) Zugabe von 3 µL einer gefilterten 1:200 Verdünnung der biologischen Probe in 0,1 % Tween 20/PBS für bis zu 9 Minuten in die jeweilige Kammer;
b) einen Waschvorgang mit 3 µL gefiltertem 0,1 % Tween 20/PBS;
c) Zugabe von 3 µL einer 1:10-Verdünnung von Quantenpunkt 525 und einer 1:100-Verdünnung von Quantenpunkt 625 für bis zu 9 Minuten;
d) einen zweiten Waschvorgang mit 3 µL gefiltertem 0,1 % Tween 20/PBS;
e) Einsetzen des Biochips in die Schublade des optischen Lesers;
f) Eingabe von Informationen, die über eine Webschnittstelle verfügbar sind; und
g) Drücken der Starttesttaste;
wobei die Vorrichtung den Biochip für bis zu 2 Minuten in ein Autofokussystem stellt, optional auf das Ende der Migration wartet, eine Bildanalyse für bis zu 2 Minuten durchführt, Ergebnisse speichert und auf der Webseite in bis zu 5 Sekunden anzeigt.

## Revendications

1. Système de détection et de surveillance de la présence d'anticorps associés à différentes étapes de la maladie de Lyme, présents dans des échantillons biologiques, comprenant
- un bouquet d'antigènes
- une biopuce
- un lecteur optique de biopuce; et
**caractérisé par le fait que**
les anticorps présents dans les échantillons biologiques sont détectés en particulier pour l'auto-immunité aigüe, chronique, et la co-infection associée à la maladie de Lyme, le bouquet d'antigènes comprenant seize antigènes, en particulier, IgG et IgM, des lysats cellulaires de E. coli, OspC, OspA, DbpA et Arp, OspE1/2, LFA1, Arp, peptides de Babesia microti et Ehrlichia, spirochètes de Borrelia burgdorferi B31, kystes de Borrelia burgdorferi B31, Borrelia garinii et Borrelia azfelii;
et en ce que
ladite micropuce comprend un premier réservoir (1) apte à contenir des échantillons biologiques, un deuxième réservoir (2) pour le tampon de lavage, un troisième réservoir (3) pour des boîtes quantiques, une zone de travail et un champ de vue (4) comprenant des vannes, des chambres de réaction et des chambres d'analyse dans lequel les chambres de réaction et d'analyse portent des revêtements d'antigènes, avec au moins une première pompe (5), une deuxième pompe (6) et neuf canaux microfluidiques (7, 8, 9, 10, 11, 12, 13, 14 et 15).

2. Système selon la revendication 1 **caractérisé par le fait que** les échantillons biologiques sont le sérum ou le fluide cérébrospinal.

3. Système selon la revendication 1 **caractérisé par le fait que** le tampon de lavage est 0,1 % Tween 20/solution PBS.

4. Système selon la revendication 1 **caractérisé par le fait que** les boîtes quantiques deviennent fluorescentes entre 400 et 900 nm, de préférence entre 500 et 700 nm, et plus préférablement à 525 nm et 625 nm, et qui sont conjuguées aux anticorps IgG anti-humain ou IgM anti-humain.

5. Système selon les revendications précédentes **caractérisé par le fait que** la zone de travail et le champ de vue (42) de la biopuce mesure jusqu'à 1 mm de longueur et jusqu'à 1 mm de largeur, de préférence 350 µm de largeur, et comprend au moins seize chambres de réaction et d'analyse.

6. Système selon les revendications précédentes **caractérisé par le fait que** chaque chambre de réaction et d'analyse comprise dans la zone de travail et le champ de vue (42) a jusqu'à 50 µm en longueur, jusqu'à 50 µm de largeur et a jusqu'à 15 µm de profondeur.

7. Système selon les revendications précédentes **caractérisé par le fait que** la biopuce comprend des pompes de fluides capillaires (43) et (44) avec un débit d'au moins 0,2 µL/minute, de préférence avec un débit de 0,4 µL/minute.

8. Système selon les revendications précédentes **caractérisé par le fait que** le lecteur optique de biopuce comprend un détecteur de fluorescence contenant une structure interne comprenant une source lumineuse, de préférence LED, une caméra, un tube d'objectif, un système d'illumination de Köhler, un système autofocus, un cadre mécanique, et un positionneur d'objectif fixe.

9. Système selon la revendication 8, **caractérisé par le fait que** le lecteur optique de biopuce contient en outre une structure intermédiaire comprenant des boucliers légers, un cadre de logement, une paroi raccordable, un raidisseur, un compartiment informatique et une structure externe comprenant le logement principal, un écran tactile, des trous de ventilation et une trappe, où le détecteur de fluorescence est conçu en particulier pour utiliser des marqueurs fluorescents à boîtes quantiques et est particulièrement adapté pour être optiquement couplé et aligné avec un système d'insertion et de retrait de la biopuce moyennant un système de serrage et le système auto-focus a un champ de vue de détection jusqu'à 1 mm de longueur, de préférence 480 µm, et jusqu'à 1 mm de largeur, de préférence 480 µm, et que le champ de détection de vue comprend le champ de vue de la biopuce (4) avec approximativement 65 µm de tolérance dans les axes X et Y pour positionner l'exactitude.

10. Système selon la revendication 9, **caractérisé par le fait que** le système de serrage du lecteur optique de biopuce comprend trois points de contact de référence avec des butées mécaniques (16) avec une position réglable et deux poussoirs (34) qui poussent la biopuce contre ces butées dans lequel outre les attaches X, Y, deux attaches élastiques maintiennent la biopuce en position le long de l'axe Z et les ressorts de retour (17) tirent les poussoirs en arrière pour permettre l'insertion et le retrait de la biopuce.

11. Système selon la revendication 9, **caractérisé par le fait que** le système d'insertion et de retrait du lecteur optique de biopuce consiste en un système à tiroir comprenant une pièce de matériau d'acier inoxydable magnétique et un mécanisme de guidage avec deux chariot de guidage (18) par rail, où dans la position fermée le tiroir est bloqué par un électroaimant (19) dans lequel le contact entre le tiroir et l'électroaimant assure un parfait alignement de l'axe X entre le détecteur et la biopuce, maintenant le détecteur isolé de la lumière, et dans la position ouverte le tiroir est bloqué avec deux aimants permanents (20) pour le maintenir en position pendant le chargement de la biopuce.

12. Procédé d'utilisation du système de détection et de surveillance de la présence d'anticorps associés à la maladie de Lyme dans des échantillons biologiques selon les revendications 1 à 11 **caractérisé par** les étapes suivantes:
a) exécution d'un algorithme dans le lecteur optique;
b) ajout de 3 µL d'une dilution filtrée 1:200 de l'échantillon biologique dans 0,1 % Tween 20/PBS pour une durée allant jusqu'à 9 minutes à la chambre respective;
c) une opération de lavage avec 3 µL de 0,1 % Tween 20/PBS filtré;
d) ajout de 3 µL d'une dilution 1:10 de boîte quantique 525 et d'une dilution 1:100 de boîte quantique 625 pour une durée allant jusqu'à 9 minutes;
e) une deuxième opération de lavage avec 3 µL de 0,1 % Tween 20/PBS filtré;
f) insertion de la biopuce dans le tiroir du lecteur optique;
g) saisie des informations disponibles depuis l'interface internet; et
h) poussée du bouton de démarrage du test, dans laquelle le dispositif place la biopuce dans un système autofocus pour une durée allant jusqu'à 2 minutes, attend optionnellement la fin de la migration, réalise l'analyse d'image pour une durée allant jusqu'à 2 minutes, stocke et affiche les résultats sur le site internet en 5 secondes maximum.

13. Procédé selon la revendication 12 **caractérisé par le fait que** les échantillons biologiques sont de préférence le sérum ou le fluide cérébrospinal.

14. Procédé d'utilisation du système des revendications 1-11, selon les revendications 12 à 13, **caractérisé par le fait que** la biopuce est insérée et retirée moyennant un système de serrage qui comprend trois points de contact de référence avec des butées mécaniques (16) avec une position réglable et deux poussoirs (34), qui poussent la biopuce contre ces butées dans lequel outre les attaches X, Y, deux attaches élastiques maintiennent la biopuce en position le long de l'axe Z et les ressorts de retour (17) tirent les poussoirs en arrière pour permettre l'insertion et le retrait de la biopuce.

15. Procédé d'utilisation du système des revendications 1-11, selon les revendications 12-14, **caractérisé par le fait que** le système de serrage fait partie d'un système à tiroir comprenant une pièce de matériau d'acier inoxydable magnétique et un mécanisme de guidage avec deux chariots de guidage (18) par rail, dans lequel la position fermée du tiroir est bloquée par un électroaimant (19) et le contact entre le tiroir et l'électroaimant assure un parfait alignement de l'axe X entre le détecteur de fluorescence et la biopuce, maintenant le détecteur de fluorescence isolé de la lumière, et dans la position ouverte le tiroir est bloqué avec deux aimants permanents (20) pour le maintenir en position pendant le chargement de la biopuce.

16. Procédé d'utilisation du système des revendications 1-11, selon les revendications 12-15, **caractérisé par le fait que** le système auto-focus comprend une étape de translation de l'axe Z avec une table guidée par roulement à billes motorisée par un servomoteur CC à balai et fonctionne moyennant trois étapes:
a) scanne une large plage de distances entre la biopuce et l'objectif, dans l'ordre de 1 mm, avec des étapes de grands mouvements dans l'ordre de 50 µm, entre chaque capture d'image, dans lequel le mouvement de l'axe Z est continu et des photos sont prises sur le champ et la distance, dans laquelle l'image est la plus nette, est enregistrée;
b) scanne précisément, avec de petits mouvements dans l'ordre de quelques microns, de préférence entre 5 et 50 µm, la plage de distance déterminée par la première étape afin de trouver la distance précise, des mouvements de l'axe Z puis une photo est obtenue lorsque l'axe Z est arrêtée et, dès que l'ensemble de la plage de la deuxième étape est scanné, l'axe Z retourne à la position correspondant à l'image la plus nette; et
c) baisse l'axe Z d'une distance correspondant à la profondeur de canaux microfluidiques de la biopuce, de préférence entre 15 et 20 µm puis baisse l'axe Z jusqu'à 10 µm pour compenser la différence optique entre le filtre de champ clair et les filtres de fluorescence.

17. Procédé d'utilisation du système des revendications 1-11, selon les revendications 12-16, **caractérisé par le fait que** l'algorithme extrait l'emplacement exact des différentes chambres de réaction dans l'image nette collectée dans la revendication 16, réalise l'analyse d'image et élabore un rapport, comprenant:
a) diminution à une image de plus faible résolution;
b) transformation de l'image du domaine temporel au domaine fréquentiel, en utilisant la transformée de Fourier;
c) corrélation de l'image fréquentielle avec elle-même moyennant l'autocorrélation;
d) inversion de la transformée de Fourier de l'image autocorrélée;
e) cartographie de l' « image Fourier inversée » en domaine pixel;
f) calcul de la déviation moyenne et standard d'intensités pixels de l'image originale de plus faible résolution;
g) calcul du facteur de corrélation entre l'image originale, telle que la déviation moyenne et standard, et les pixels de l' « image Fourier inversée »;
h) calcul du score sur la base du minimum du facteur de corrélation de l'étape précédente;
i) localisation du champ de vue, sa position et orientation moyennant le filtrage par motif, dans lequel les motifs sont créés en dessinant 16 régions d'intérêt alignées avec le motif, ces dernières étant environ 16 régions correspondant aux zones où les données sont utilisées pour fournir des résultats du test de diagnostic; et
j) l'algorithme de filtrage de motif est utilisé avec le motif stocké de l'étape précédente;
dans lequel le nouvel algorithme d'analyse d'image fonctionne en 4 étapes consécutives, appelées détermination du meilleur temps d'exposition de caméra, détermination de résidus et le retrait de ces derniers, détermination de fluorescence de fond et analyse de chambres de réaction sans résidus et fluorescence de fond.

18. Procédé d'utilisation du système des revendications 1-11, selon les revendications 12-17, **caractérisé par le fait qu'**il comprend l'exécution d'un algorithme personnalisé dans le lecteur optique et:
a) ajout de 3 µL d'une dilution filtrée 1:200 de l'échantillon biologique dans 0,1 % Tween 20/PBS pour une durée allant jusqu'à 9 minutes à la chambre respective;
b) une opération de lavage avec 3 µL de 0,1 % Tween 20/PBS filtré;
c) ajout de 3 µL d'une dilution 1:10 de boîte quantique 525 et une dilution 1:100 de boîte quantique 625 pour une durée allant jusqu'à 9 minutes;
d) une deuxième opération de lavage avec 3 µL de 0,1 % Tween 20/PBS filtré;
e) insertion de la biopuce dans le tiroir du lecteur optique;
f) saisie des informations disponibles depuis une interface internet; et
g) poussée du bouton de démarrage du test;
dans laquelle le dispositif place la biopuce dans un système autofocus pour une durée allant jusqu'à 2 minutes, attend optionnellement la fin de la migration, réalise l'analyse d'image pour une durée allant jusqu'à 2 minutes, stocke et affiche les résultats sur le site internet en 5 secondes maximum.
